Europäisches Patentamt
European Patent Office
Office européen des brevets

(11) Publication number: **0 097 628 B1**

# (12) EUROPEAN PATENT SPECIFICATION

(45) Date of publication of patent specification: **19.09.90**

(51) Int. Cl.[5]: **C 07 D 211/22,** C 07 D 211/26, A 61 K 31/445

(21) Application number: **83850084.1**

(22) Date of filing: **28.03.83**

## (54) Piperidine derivatives, processes for their preparation and pharmaceutical preparation containing them.

(30) Priority: **30.03.82 SE 8202023**

(43) Date of publication of application: **04.01.84 Bulletin 84/01**

(45) Publication of the grant of the patent: **19.09.90 Bulletin 90/38**

(84) Designated Contracting States: **AT BE CH DE FR GB IT LI LU NL SE**

(56) References cited:
**EP-A-0 030 526**

**Medical Chemistry, third edition, part I, A. Burger, Wiley-Interscience, New York, 1970, pages 81-87**

**E. Schröder and al., Arzneimittelchemie I, Georg Thieme Verlage Stuttgart 1976, pages 46-49**

**E. Mutschler, Arzneimittelwirkungen, Wissenschaftliche Verlagsgesellschaft, Stuttgart, 1981, page 57**

(73) Proprietor: **Carlsson, Per Arvid Emil**
**Torild Wulffsgatan 50**
**S-413 19 Göteborg (SE)**

(72) Inventor: **Arvidsson, Folke Lars-Erik**
**Seminariegränd 3**
**S-752 28 Uppsala (SE)**
Inventor: **Carlsson, Per Arvid Emil**
**Torild Wulffsgatan 50**
**S-413 19 Göteborg (SE)**
Inventor: **Hacksell, Uli Alf**
**1737 Schimer Avenue Appartment 12**
**Bethlemen Pennsylvania (US)**
Inventor: **Hjorth, John Stephan Mikael**
**Blavalsgatan 7A**
**S-414 75 Göteborg (SE)**
Inventor: **Johansson, Anette Margareta**
**Blodstensvägen 7**
**S-752 44 Uppsala (SE)**
Inventor: **Lindberg, Per Lennart**
**Knapehall 64**
**S-436 00 Askim (SE)**
Inventor: **Nilsson, John Lars Gunnar**
**Tullinge Strand 3OB**
**S-146 00 Tullinge (SE)**
Inventor: **Sanchez, Domingo**
**Snipasvägen 24**
**S-448 00 Floda (SE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 097 628 B1

(72) Inventor: **Svensson, Kjell Anders Ivan**
**Dr. Bex Gata 2**
**S-413 24 Göteborg (SE)**
Inventor: **Wikström, Hakan Vilhelm**
**Gibsons väg 25**
**S-433 61 Partille (SE)**

(74) Representative: **Perry, Robert Edward et al**
**GILL JENNINGS & EVERY 53-64 Chancery Lane**
**London WC2A 1HN (GB)**

**Description**

The present invention relates to piperidine derivatives and to their use in therapy of the central nervous system.

EP—A—0030526 discloses, inter alia, compounds of formula I,

wherein Q is H, $R^1CO$—, $R^2R^3N$—CO—, allyl or benzyl;

wherein $R^1$ is $C_{1-5}$ alkyl or optionally substituted phenyl;

$R^2$ is $C_{1-5}$ alkyl, phenethyl, benzyl or phenyl; and

$R^3$ is H or $C_{1-5}$ alkyl;

and pharmaceutically-acceptable acid addition salts thereof. These compounds are potent neuropharmacological agents. Thus, these compounds are active as presynaptic dopamine receptor agonists when administered to animals including man. These compounds are thus useful for treatment of disorders in the central nervous system, especially psychotic disorders in man.

EP—A—0030526 refers to and covers in general the pure enantiomers as well as mixtures thereof. However, the enantiomers are not specifically disclosed.

A novel compound according to the present invention is a pure enantiomer of formula I, wherein Q is as defined above, and

wherein $R^1$ is $C_{1-17}$ aliphatic hydrocarbyl, phenyl, 2,6-dimethylphenyl, 3- or 4-hydroxyphenyl, 3- or 4-($C_{2-7}$ alkanoyloxy)phenyl, or —$CHR^6$—$NR^7R^8$ wherein $R^6$ is H, $C_{1-5}$ alkyl or phenyl, $R^7$ is H, $C_{1-5}$ alkyl, formyl, acetyl, benzoyl, methoxycarbonyl, phenoxycarbonyl or benzyloxycarbonyl, and $R^8$ is H or $C_{1-5}$ alkyl; and

either $R^2$ is H, $C_{1-5}$ alkyl, or a phenethyl, benzyl or phenyl group which may be mono- or di-ring-substituted by methyl, methoxy, hydroxy, nitro, cyano or a halogen atom, and $R^3$ is H, $C_{1-5}$ alkyl or phenyl, or $NR^2R^3$ is a 5, 6 or 7-membered ring that may contain 1 to 3 double bonds and/or 1 or 2 further heteroatoms selected from N, O and S;

said enantiomer having the same absolute configuration at the asymmetric carbon atom (*) as that of the (−)-enantiomer of the compound of formula I wherein Q is H;

or a pharmaceutically-acceptable acid addition salt thereof.

The novel compounds possess unexpected valuable therapeutical properties in addition to those previously described. In particular, they have utility in the manufacture of medicaments having post-synaptic dopamine antagonist activity.

The (−)-enantiomer of the compound of formula I wherein Q is H is the laevo rotameric form. By X-ray crystallography, the absolute configuration of the (−)-enantiomer of the compound of formula I wherein Q is H has been determined to be the S configuration. Thus, the compounds of the invention according to formula I all have S configuration. Compounds of the invention having Q other than H have the same absolute configuration as said (−)-enantiomer, it being understood that said latter compounds are not necessarily the (−) or laevo rotameric forms.

Any alkyl group may be straight or branched. Any aliphatic hydrocarbon residue $R^1$ may be saturated or unsaturated. Symbols for numbers, atoms or groups referred to below have the broadest meaning previously assigned unless otherwise specified.

Both organic and inorganic acids can be employed to form non-toxic pharmaceutically-acceptable acid addition salts of the compounds of this invention. Illustrative acids are sulphuric, nitric, phosphoric, hydrochloric, citric, acetic, lactic, tartaric, pamoic, ethanedisulphonic, sulphamic, succinic, cyclohexylsulphamic, fumaric, maleic and benzoic acid. These salts are readily prepared by methods known in the art.

In the pure enantiomers of the invention, Q is in particular H, $R^1CO$, $R^2R^3NCO$—, allyl or benzyl, wherein $R^1$ is $C_{1-5}$ alkyl, phenyl, 2,6-dimethylphenyl, 3- or 4-hydroxyphenyl or 3- or 4-alkanoyloxyphenyl, $R^2$ is $C_{1-5}$ alkyl, phenethyl, benzyl or phenyl, and $R^3$ is H or $C_{1-5}$ alkyl.

More preferably, Q is H, $R^1CO$ or $R^2R^3NCO$.

Compounds to be specifically mentioned are:

(−)-3-(3-hydroxyphenyl)-1-propylpiperidine,
(−)-3-[3-(4-pivaloyloxybenzoyloxy)phenyl]-1-propylpiperidine,
(−)-3-(3-allyloxyphenyl)-1-propylpiperidine,
(−)-3-(3-decanoyloxyphenyl)-1-propylpiperidine,
S-3-(3-pivaloyloxyphenyl)-1-propylpiperidine,
S-3-(3-acetoxyphenyl)-1-propylpiperidine,
S-3-(3-benzyloxyphenyl)-1-propylpiperidine,
S-3-[3-(N,N-dimethylcarbamoyloxy)phenyl]-1-propylpiperidine,
S-3-[3-(N-phenylcarbamoyloxy)phenyl]-1-propylpiperidine, and
S-3-[3-(N-benzyloxycarbonylalanyl)phenyl]-1-propylpiperidine.

The invention takes into consideration that compounds which differ structurally from formula I, after administration to a living organism, may be transformed to a compound of the formula I and, in this transformed structural form, exert their effects. This consideration is a further aspect of the invention. Likewise, certain compounds of formula I may be metabolised into other compounds of formula I before exerting their effect. Compounds of the invention wherein Q is $R^1CO$, $R^2R^3NCO$, allyl or benzyl are believed to exert their main activity after metabolism to the compound wherein Q is H.

Pure enantiomers of the invention may be prepared, starting from the corresponding pure enantiomers, by any of methods (a), (b), (c), (d), (e) and (i) as described in EP—A—0030526 (when R = propyl, n = 2).

Another method, for preparing a compound of the invention when Q is H, comprises reducing a pure enantiomer of formula II, wherein $R^e$ is H or benzyl, having a 2,3- or 3,4-double bond. The reduction is preferably carried out by catalytic hydrogenation with an appropriate chiral homogeneous phase catalyst such as a Rh-complex with chiral phosphines. If required, the product may be purified to obtain only the desired enantiomer in a pure form.

A racemic mixture, or a mixture partly enriched in one of the enantiomers, of a compound of formula I may be subjected to enantiomeric separation to obtain the desired enantiomer of formula I. This may be done by methods known in the art. These methods include recrystallisation of diastereoisomeric salts with pure enantiomers of acids such as tartaric acid, *O,O'*-dibenzoyltartaric acid, mandelic acid and camphor-10-sulphonic acid.

Free bases formed may subsequently be converted into their acid addition salts, and acid addition salts formed may subsequently be converted into the corresponding bases or other acid addition salts.

Starting materials for the methods of preparation described above may be obtained by several methods known in the art, in particular in EP—1—0030526, and/or described below.

The enantiomeric starting material for method (a) according to EP—A—0030526 (i.e. of formula III: Z = $OR^a$, $R^d$ = $C_{1-5}$ alkyl or $C_{2-6}$ acyl, R = propyl) may be prepared by the following method:

A compound of formula III (Z as defined above, R = H: racemic mixture) is resolved either by first conversion into the N-benzyl analogue (R = benzyl) followed by recrystallisation of the (+)-tartaric acid salt and debenzylation by hydrogenation, or (when $R^a$ is alkyl) by first conversion into the (−)-O-methylmandelic acid amide (R = 2-phenyl-2-methoxyethanoyl) followed by chromatographic separation of the two diastereomers and cleavage by $KOBu^t$ in tetrahydrofuran with traces of water. The enantiomer with the desired absolute configuration (R = H) is then alkylated by propionylation followed by $LiAlH_4$ reduction to the desired compound having the appropriate absolute configuration at the asymmetric carbon atom.

The enantiomeric starting material for method b) of EP—A—0030526 may be prepared by the following method: the propyl group is introduced into 3-phenylpiperidine (III: Z = OH, R = H) as previously described with previous or subsequent resolution into the desired enantiomer whereupon the compound is treated with $Cl_2$, $H_2SO_4$ or $HNO_3$ followed by reduction to the formation of an isomeric mixture from which the desired compound (III: Z = Cl, $SO_3H$ or $NH_2$, R = propyl) is obtained by chromatographic separation.

An enantiomeric starting material for method e) of EP—A—0030526 may be prepared by the following method:

A compound of formula III (Z = $OR^a$, R = $COC_2H_5$) may be formed by N-acylation of a corresponding compound of formula III (R = H), preparable as described above, with $C_2H_5COCl$ in the presence of a base. The ether function is then cleaved with $BBr_3$ to give the desired compound (III: Z = OH, R = $COC_2H_5$). If desired, the hydroxy group may then be acylated with an acyl chloride to form the corresponding compound wherein Z is $R^1COO$, or alkylated with an allyl or benzyl halide to form the corresponding compound wherein Z is allyloxy or benzyloxy.

The starting material of formula II may be prepared by the following method: (3-hydroxyphenyl)magnesium bromide or (3-benzyloxyphenyl)magnesium bromide is reacted with 1-propylpiperidin-3-one, followed by elimination of the 3-hydroxy group from the resultant 3-hydroxy-3-(3-hydroxyphenyl)-1-propylpiperidine or 3-benzyloxyphenyl analogue.

Pharmaceutical preparations of the compounds of the invention constitute a further aspect of the invention. For such preparations reference is made to pages 23 to 25 of EP—A—0030526.

In therapeutic treatment, the suitable daily doses of the compounds of the invention are 200—10,000 mg for oral application, preferably 1,000—6,000 mg, and 1—1,000 mg for parenteral application, preferably 50—500 mg.

## WORKING EXAMPLES

The following examples will further illustrate the invention. Temperatures are in °C.

### Preparation of Intermediates

#### Example I1

(+)-3-(3-Methoxyphenyl)piperidine hydrochloride (Method A)

(+)-Dibenzoyl-D-tartaric acid (28.2 g, 0.075 mol) in hot methanol (350 ml) was added to N-benzyl-3-(3-methoxyphenyl)piperidine (21.1 g, 0.075 mol) in hot methanol (100 ml). After two days the salt that separated was recrystallized three times from methanol. The collected salt (8.3 g) was treated with 1 M NaOH (250 ml) and the free amine was extracted with ether (3 × 150 ml). The combined ether layers were dried ($K_2CO_3$) and the solvent evaporated. The residual amine was then passed through a short alumina column with ether as eluant and then converted to the hydrochloride. One recrystallization from methanol-ether gave (−)-N-benzyl-3-(3-methoxyphenyl)-piperidine hydrochloride (3.8 g), m.p. 164—165°C; $[\alpha]_D^{22}$ −43.1° (C 2.1, $CH_3OH$).

(−)-N-benzyl-3-(3-methoxyphenyl)piperidine hydrochloride (3.8 g, 0.0120 mol) was dissolved in ethanol (80 ml), 10% Pd/C was added and the mixture was hydrogenated at room temperature and

atmospheric pressure (28 h). The catalyst was removed (Celite) by filtration, the solvent was evaporated off and the crystalline residue was recrystallized from methanol-ether giving the desired (+)-3-(3-methoxyphenyl)-piperidine hydrochloride (2.54 g, 30% total yield of the maximal theoretical) m.p. 175.5—177°C; $[\alpha]_D^{22}$ +10.1° (C 2.1, $CH_3OH$).

Example I2

(+)-3-(3-Methoxyphenyl)piperidine hydrochloride (Method A)

R-(−)-α-Methoxyphenylacetic acid (11.0 g, 0.066 mol) and $SOCl_2$ (85 ml) was mixed under ice-cooling and the mixture was stirred at 20° for 2 h. Excess of $SOCl_2$ was evaporated off (at 20°) and the residual acid chloride oil was dissolved in $CH_2Cl_2$. The solution was added at 20° to a stirred mixture of 3-(3-methoxyphenyl)piperidine hydrochloride (15.1 g, 0.066 mol), $CH_2Cl_2$ (280 ml) and 2.5% aqueous NaOH (560 ml). After 10 min. stirring the phases were separated and the organic phase was washed once with water and dried ($Na_2SO_4$). Filtration and evaporation of the solvent gave 1-(R-α-methoxyphenylacetyl)-3-(3-methoxy-phenyl)piperidine as a crude oil (21.8 g).

The crude oil of 1-(R-α-methoxyphenylacetyl)-3-(3-methoxyphenyl)-piperidine (21.8 g) was chromatographed on a $SiO_2$ column (600 g $SiO_2$) with light petroleum-ether (starting with 50:50 mixture and successive increasing the ether content to 100%) as eluant. The fractions containing that one of the two diastereomers, which is eluated first, in nearly pure form were combined and the solvent evaporated off giving the desired diastereomeric amide as an oil (7.7 g, 0.023 mmol) (containing 0.5% of the other diastereomer according to HPLC). This was dissolved in dry tetrahydrofuran (400 ml) and potassium-tert-butoxide (16.1 g, 0.144 mol) and water (1.33 g, 0.074 mol) was added under stirring at room temperature. The mixture was stirred at this temperature over night and then the mixture was partitioned between ether and water. After drying ($Na_2SO_4$) of the organic phase excess of HCl-saturated ethanol was added and the solvent was evaporated off. The residue was redissolved twice in absolute ethanol and the solvent evaporated, giving a crystalline residue. Recrystallization from ethanol-ether gave the desired (+)-3-(3-methoxyphenyl)piperidine hydrochloride (4,0 g, 53% total yield of the maximal theoretical), m.p. 175.5—177°C; $[\alpha]_D^{22}$ = +10.1° (C 2.1, $CH_3OH$).

Example I3

(−)-3-(3-Methoxyphenyl)-N-n-propylpiperidine hydrochloride (Method A, E and e)

(+)-3-(3-Methoxyphenyl)piperidine hydrochloride (1.5 g, 0.0066 mol) was treated with 2N NaOH (50 ml) and the free amine was extracted with ether. After drying ($K_2CO_3$), filtering and evaporation the residual free amine oil (1.2 g) was dissolved in dry ether (50 ml) and the triethylamine (1.4 ml, 0.0010 mol) was added. Then propionyl chloride (0.87 ml, 0.0010 mol) dissolved in dry ether (5 ml) was slowly added at 0° under stirring, and the mixture was stirred at room temperature for 30 min. The precipitate was filtered off and the solvent was evaporated giving an oily residue. This residue was dissolved in dry tetrahydrofuran (50 ml) and added to a suspension of $LiAlH_4$ (0.75 g, 0.0020 mol) in dry tetrahydrofuran (75 ml) under $N_2$. After refluxing for 4 h. the mixture was hydrolysed, the precipitate was filtered off and the solvent was evaporated. The residue was dissolved in ether and passed through an alumina column with ether as eluant. Addition of HCl-saturated ethanol, evaporation of the solvent and recrystallization gave (−)-3-(methoxyphenyl)-N-n-propylpiperidine hydrochloride (1.48 g, 83%), 200,5—202°C; $[\alpha]_D^{22}$ −6.7 (C 2.1, $CH_3OH$).

Example I4

(−)-3-(3-methoxyphenyl)-N-n-propyl piperidine hydrochloride (Method A and d)

$NaBH_4$ (0.61 g, 0.016 mol) was added portionwise under stirring to a solution of propionic acid (3.8 g, 0.051 mol) in dry benzene (15 ml). The temperature was kept below 15°C for 2 h and then (+)-3-(3-methoxyphenyl)-piperidine (0.61 g, 0.0032 mol) dissolved in dry benzene (10 ml) was added and the mixture was refluxed for 3 h. The reaction mixture was allowed to reach room temperature and was then extracted with 2.5 M NaOH (20 ml). The aqueous phase was extracted with benzene, all the benzene phases mixed, dried ($Na_2SO_4$) and the solvent evaporated giving an oily residue. The product was precipitated as hydrochloride and recrystallized from methanol/ether, yielding the pure product (0.60 g, 73%), mp. 200—202°C.

Preparation of End Compounds

Example E1

(−)-3-(3-Hydroxyphenyl)-N-n-propylpiperidine hydrochloride (Method a)

(−)-(3-Methoxyphenyl)-N-n-propylpiperidine hydrochloride (1.20 g, 0.0044 mol) in 48% aqueous HBr was heated at 120° for 4 h under $N_2$. The volatiles were removed *in vacuo* and the residue was recrystallized from methanol-ether giving (−)-3-(3-hydroxyphenyl)-N-n-propylpiperidine hydrobromide (1.10 g) mp. 166—167°C; $[\alpha]_D^{22}$ −5.8 (C 2.0, $CH_3OH$). The hydrobromide was alkalinized with saturated $NaHCO_3$ (25 ml) and the mixture was extracted with ether (4 × 20 ml). The combined ether layers were dried ($Na_2SO_4$), filtered and HCl-saturated ether was added giving a precipitate which was recrystallized giving (−)-3-(3-hydroxyphenyl)-N-n-propylpiperidine hydrochloride (0.84 g, 75%), mp. 187—188°C; $[\alpha]_D^{22}$ −7.1 (C 2.2, $CH_3OH$).

Example E2

(−)-3-(3-Hydroxyphenyl)-N-n-propylpiperidine hydrochloride (Method a)

(−)-3-(3-Methoxyphenyl)-N-n-propylpiperidine (1.50 g, 0.0064 mol) was dissolved in $CH_2Cl_2$ (25 ml) and cooled to −78°. $BBr_3$ (3.0 ml, 0.031 mol) was added under stirring. The cooling medium was removed and the mixture was allowed to reach room temperature. Alkalinization with excess 10% $Na_2CO_3$, extraction with $CH_2Cl_2$, drying ($Na_2SO_4$) and evaporation of the solvent gave a yellow oil (1.5 g). The oil was dissolved in abs. ethanol and excess of HCl-saturated ethanol was added. Evaporation to dryness followed by recrystallization from ether-ethanol gave (−)-3-(3-hydroxyphenyl)-N-n-propylpiperidine hydrochloride (1.05 g, 64%), mp. 187—188°C.

Example E3

(−)N-n-Propyl-3-[3-(4-pivaloyloxybenzoyloxy)phenyl]piperidine hydrochloride (Method c)

(−)N-n-propyl-3-(3-hydroxyphenyl)piperidine hydrochloride (0.400 g, 1.56 mmol) was suspended in dry dichloromethane (4 ml). Pivaloyloxybenzoylchloride (0.413 g, 1.72 mmol) was dissolved in a mixture of dichloromethane (4 ml) and pyridine (0.136 g, 1.72 mmol). The solution was added to the suspension and the mixture was refluxed for 20 hours. The clear solution was cooled, washed with aqueous $NaHCO_3$ and dried with $Na_2SO_4$. After evaporation the residual oil was dissolved in ethanol, one equivalent HCl-saturated ethanol was added and the product precipitated by adding ether. Filtering and drying yielded 360 mg (50%) of the pure desired hydrochloride. Mp. 228—229°C $[\alpha]_D^{20}$ (C = 1.5, MeOH) (−)9.05°.

Example E4

(−)N-n-Propyl-3-(3-allyloxyphenyl)piperidine hydrochloride (Method c)

(−)N-n-propyl-3-(3-hydroxyphenyl)piperidine hydrochloride (0.255 g, 1 mmol) and tetrabutylammonium hydrogen sulphate (0.340 g, 1 mmol) were suspended in dichloromethane (2 ml) and allylbromide (0.133 g, 1.1 mmol). 2-M NaOH (2 ml) was added during two minutes and the mixture was stirred at room temperature for half an hour. The layers were separated and the organic layer was dried over $Na_2SO_4$. After removal of the solvent by distillation, the residual oil was dissolved in ether (50 ml). A theoretical quantity of tetrabutylammonium iodide was precipitated. The precipitate was filtered off and to the filtrate was added one equivalent HCl-saturated ether. White crystals of the desired compound was obtained. The crystals were filtered, washed on the filters with ether and dried. Yield 190 mg (65%). Mp. 136—138°C $[\alpha]_D^{20}$ (C = 1.5 MeOH) (−)7.6°.

Example E5

(−)-N-n-Propyl-3-(3-hydroxyphenyl)piperidine hydrochloride (Method b)

To a solution of the appropriate enantiomeric form of N-n-propyl-3-(3-aminophenyl)piperidine (0.27 g, 0.0013 mol) in 6 M $H_2SO_4$ (1 ml) $NaNO_2$ (0.077 g, 0.0011 mol) dissolved in water (0.2 ml) was added dropwise at 5°C and then the mixture was stirred at 5°C for 1 h. The resulting mixture was added dropwise to refluxing 10% $H_2SO_4$ (1.5 ml) and the reflux was continued for 5 min. Cooling, alkalising ($Na_2CO_3$), extraction with ether, drying and evaporation of the organic phase gave the desired product as a free base. Conversion to the hydrochloride followed by recrystallization gave 0.059 g (20%) of (−)-N-n-propyl-3-(3-hydroxyphenyl)piperidine hydrochloride, m.p. 176—177°C.

Example E6

(−)-N-n-Propyl-3-(3-allyloxyphenyl)piperidine hydrochloride (Method e)

A solution of the appropriate enantiomeric form of N-n-propionyl-3-(3-allyloxyphenyl)piperidine (0.04 g, 0.16 mmol) in dried ether (5 ml) was dropped to a suspension of $LiAlH_4$ (0.04 g) in dried ether under nitrogen and stirring, and the mixture was refluxed for 30 min. $H_2O$ (0.04 ml), 15% NaOH (0.04 ml) and $H_2O$ (0.2 ml) were added and the precipitated crystals were filtered off and washed with ether. The solution was dried with $Na_2SO_4$. Evaporation to dryness gave an oily residue which was dissolved in ether. Addition of HCl-saturated ether resulted in precipitation of white crystals. The crystals were centrifugated and treated with light petroleum, centrifugated and dried. Yield 0.02 g of the end compound having physical properties in accordance with Example E4.

Example E7

(−)-3-(3-Decanoyloxyphenyl)-1-propylpiperidine hydrochloride

(−)-3-(3-hydroxyphenyl)-1-propylpiperidine hydrochloride (1.5 g, 5.86 mmol) was suspended in dry dichloromethane (15 ml). Decanoylchloride (1.2 g, 6.29 mmol) was dissolved in a mixture of dichloromethane (15 ml) and pyridine (0.5 g, 6.32 mmol). The solution was added to the suspension and the mixture was refluxed for 20 hours. The clear solution was cooled, washed with aqueous $NaHCO_3$ and dried with $Na_2SO_4$. After evaporation the residual oil was dissolved in ether and precipitated as hydrochloride salt with one equivalent HCl in ether. The salt was recrystallized twice from aceton. Yield: 0.7 g (30%). M.p. 142—144°C. $[\alpha]_D^{20}$ (C = 1.8, MeOH) = −5.6°. MS(70 eV): m/z 373 (5%), 344 (100%), 190 (7%).

Example E8

(−)-3-(3-Phenylcarbamoyloxyphenyl)-1-propylpiperidine

The title compound was prepared by esterification of (−)-3-(3-hydroxyphenyl)-1-propylpiperidine. [α] = −8.0°. M.p. (HCl) 194—195°C.

The following examples illustrate how the compounds of the present invention may be included into pharmaceutical preparations.

Example P1

Preparation of soft gelatine capsules

500 g of active substance are mixed with 500 g of corn oil, whereupon the mixture is filled in soft gelatine capsules, each capsule containing 100 mg of the mixture (i.e. 50 mg of active substance).

Example P2

Preparation of tablets

0.5 kg of active substance are mixed with 0.2 kg of silicic acid of the trade mark Aerosil. 0.45 kg of potato starch and 0.5 kg of lactose are mixed therewith and the mixture is moistened with a starch paste prepared from 50 g of potato starch and distilled water, whereupon the mixture is granulated through a sieve. The granulate is dried and sieved, whereupon 20 g of magnesium stearate are mixed into it. Finally the mixture is pressed into tablets each weighing 172 mg.

Example P3

Preparation of a syrup

100 g of active substance are dissolved in 300 g of 95% ethanol, whereupon 300 g of glycerol, aroma and colouring agents (q.s.) and 1000 ml of water are mixed therein. A syrup is obtained.

Example P4

Preparation of an injection solution

Active substance (hydrochloride) (1 g), sodiumchloride (0.8 g) and ascorbic acid (0.1 g) are dissolved in sufficient amount of distilled water to give 100 ml of solution. This solution, which contains 10 mg of active substance per ml, is used in filling ampoules, which are sterilized by heating at 120°C for 20 minutes.

Pharmacological evaluation

Among the compounds described in EP—A1—0030526, the compound N-n-propyl-3-(3-hydroxyphenyl)-piperidine, hereinafter referred to as 3-PPP, was presented in greatest detail.

It was shown that 3-PPP and its congeners are capable of inhibiting the physiological activity of central dopaminergic neurons by activating a subpopulation of dopaminergic receptors presumably located on the dopaminergic neuron itself, i.e. presynaptic receptors or autoreceptors. The effect proved to be selective, in that no concomitant activation of the classical postsynaptic dopaminergic receptors could be detected.

These observations were made on racemic mixtures. The two enantiomers of 3-PPP have now been tested separately. Surprisingly the levorotatory enantiomer, (−)-3-PPP, not only was capable of activating the presynaptic dopaminergic receptors (autoreceptors) but concomitantly *reduced* the sensitivity of the postsynaptic dopaminergic receptors to dopaminergic agonists. By the combined activation of presynaptic receptors and partial blockade of postsynaptic receptors the inhibitory action on dopaminergic neurotransmission will be stronger than by either effect alone. In other words, the levorotatory enantiomer is superior to the racemic mixture as an inhibitor of dopaminergic neurotransmission by acting both presynaptically as an agonist and postsynaptically as an antagonist.

The reason for the difference in pharmacological profile between the levorotatory enantiomer and the racemic mixture has been clarified: the dextrorotatory enantiomer was found to be a dopaminergic receptor agonist by activating both the presynaptic and the postsynaptic receptors. Thus, the two enantiomers antagonize each other on the postsynaptic dopaminergic receptor. As a consequence, the racemic mixture will be active only on the presynaptic receptor (autoreceptor). The pharmacological tests demonstrating the properties of the two enantiomers of 3-PPP are presented below.

1. *Evidence for presynaptic dopaminergic receptor (autoreceptor) agonist activity of both enantiomers of 3-PPP*

Both enantiomers of 3-PPP cause a dose-dependent decrease in spontaneous exploratory motor activity of rats. In male Sprague-Dawley rats injected subcutaneously with either enantiomer of 3-PPP and 5 min later placed in Motron boxes for measurement of motor activity the number of counts during the first 30 min was recorded at doses between 0.053 and 64 mg/kg. Both enantiomers cause a decrease of locomotor activity to 30—40% of control. However, the (+)-enantiomer causes a decrease only after doses between 0.25 and 4 mg/kg s.c. After 8 mg/kg there is no effect, and after 64 mg/kg there is a significant increase to more than 200% of control. In contrast, the (−)-enantiomer causes a decrease after all these doses, with no reversal of the effect after larger doses.

In the dopaminergic neurotransmission L-tyrosine is hydroxylated to L-dopa, which is decarboxylated to the transmitter substance dopamine. After inhibition of the aromatic amino acid decarboxylase by

means of 3-hydroxybenzylhydrazine HCl (NSD 1015) both enantiomers of 3-PPP cause a dose-dependent decrease at doses between 0.053 and 64 mg/kg in the formation of dopa in dopamine-rich brain regions of rats pretreated with reserpine or gamma-butyrolactone (GBL). Pretreatment with GBL and probably also reserpine precludes the influence of postsynaptic dopamine receptors on dopa formation and thus provides the opportunity to study the influence of presynaptic autoreceptors separately (cf. Hjorth et al., Life Sci. Vol 28, pp. 1225—1238, 1981). The effect of both enantiomers on dopa formation is blocked by the dopamine-receptor antagonist haloperidol. These findings provide strong evidence for stimulation of presynaptic dopamine receptors (autoreceptors).

In rats not pretreated with GBL or reserpine the two enantiomers act differently on dopa formation at doses between 0.053 and 64 mg/kg: the (+)-form causes a decrease, whereas the (−)-form causes an increase in dopa formation in the striatum and no change in the limbic region. These changes in dopa formation are matched by corresponding changes in the levels of the dopamine metabolites 3,4-dihydroxyphenyl acetic acid and homovanillic acid. This differential action of the two enantiomers will be commented below.

2. *Evidence for postsynaptic dopaminergic receptor agonist activity of (+)-3-PPP*

In reserpine-treated rats (+)-3-PPP causes a marked stimulation of locomotor activity at doses between 0.25 and 64 mg/kg. The effect is blocked by haloperidol. These findings provide strong evidence for a stimulating action of (+)-3-PPP on postsynaptic dopaminergic receptors. The (−)-form causes only a very weak stimulation of motor activity in reserpine-pretreated rats, indicating virtual absence of stimulating action on postsynaptic dopaminergic receptors.

3. *Evidence for postsynaptic dopaminergic receptor antagonist activity of (−)-3-PPP*

The locomotor stimulating action of the dopamine receptor agonist apomorphine is antagonized by (−)-3-PPP. This effect is clearcut after a dose of 8 mg/kg s.c. of (−)-3-PPP, but not after 2 mg/kg. Thus the postsynaptic receptor antagonist activity requires a larger dose than the presynaptic receptor agonist activity, which is evident after doses down to 0.25 mg/kg. The above-mentioned ability of (−)-3-PPP to stimulate dope formation and to raise dopamine-metabolite levels in rat striatum provides further evidence for blockade of postsynaptic dopamine receptors, resulting in feedback-mediated stimulation of dopaminergic neurons.

Further, (−)-3-PPP and racemic 3-PPP have been compared regarding antagonism of apomorphine-induced locomotor stimulation. Either form of 3-PPP was injected subcutaneously in a dose of 8 mg/kg 20 min and apomorphine in a dose of 1 mg/kg subcutaneously 5 min before placing the rats in the Motron for 30 min. Shown are the counts per 30 min, means ± s.e.m. and number of experiments (n). As demonstrated in Table 1 (−)-3-PPP antagonized said stimulation while the racemate did not.

The (−)-form of 3-PPP has a striking antagonistic action against the locomotor stimulating activity of (+)-amphetamine. This effect is probably the result of the simultaneous stimulation of presynaptic dopaminergic receptors and partial blockade of postsynaptic dopaminergic receptors. All the major antipsychotic agents in current use are potent amphetamine antagonists, and such activity is considered to be predictive of antipsychotic action.

Conclusion

The compounds under consideration are centrally acting selective dopamine autoreceptor stimulating agents, and thus of great clinical interest in the treatment of psychotic disorders such as schizophrenia and a number of other disease states such as tardive dyskinesia, Huntington's chorea, alcoholism and drug abuse, said psychotic disorders and other disease states possibly being associated with a pathological increase in central dopamine transmission. Extrapyramidal motor disturbances of choreatic type are avoided with the compounds of the invention. As compared with the known racemates, the pure enantiomers of the invention have a better efficacy in the suggested treatment in having an unexpected postsynaptic dopamine antagonist activity in addition the presynaptic dopamine agonist activity.

TABLE 1

Antagonism of Apomorphine-Induced Locomotor Stimulation

| | | |
|---|---|---|
| Vehicle (control) | 174 ± 15 | (10) |
| Apomorphine | 513 ± 35 | (10)[a] |
| (−)-3-PPP + Apomorphine | 276 ± 50 | (5)[b] |
| (±)-3-PPP + Apomorphine | 443 ± 26 | (5)[c] |

[a] differs from control, $p < 0.001$

[b] differs from apomorphine, $p < 0.005$

[c] not significantly different from apomorphine; differs from control, $p < 0.001$

Best Mode of Carrying Out the Invention

The compound (−)-N-n-propyl-3-(3-hydroxyphenyl)piperidine and its salts and processes for preparing said compound represent the best mode of carrying out the invention.

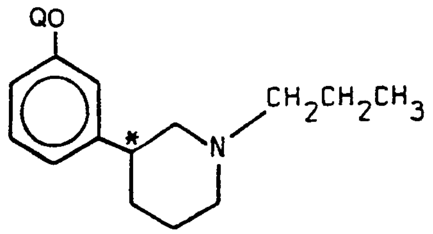

I

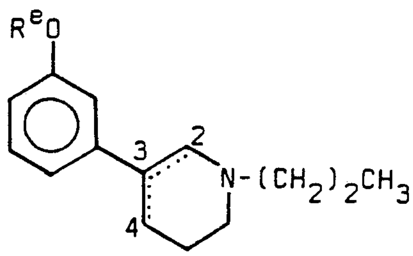

II

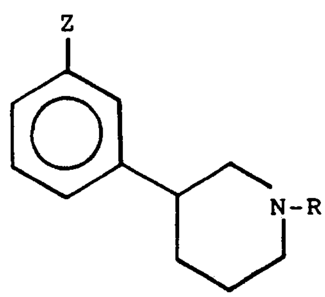

III

**Claims for the Contracting States: BE CH DE FR GB IT LU NL SE**

1. A pure enantiomer of a compound of formula I

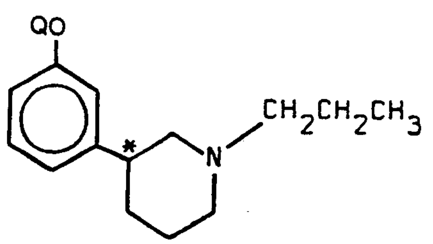

I

wherein Q is H, $R^1CO$—, $R^2R^3N$—CO—, allyl or benzyl;

wherein $R^1$ is $C_{1-17}$ aliphatic hydrocarbyl, phenyl, 2,6-dimethylphenyl, 3- or 4-hydroxyphenyl, 3- or 4-($C_{2-7}$ alkanoyloxy)phenyl, or —$CHR^6$—$NR^7R^8$ wherein $R^6$ is H, $C_{1-5}$ alkyl or phenyl, $R^7$ is H, $C_{1-5}$ alkyl, formyl, acetyl, benzoyl, methoxycarbonyl, phenoxycarbonyl or benzyloxycarbonyl, and $R^8$ is H or $C_{1-5}$ alkyl; and

either $R^2$ is H, $C_{1-5}$ alkyl, or a phenethyl, benzyl or phenyl group which may be mono- or di-ring-substituted by methyl, methoxy, hydroxy, nitro, cyano or a halogen atom, and $R^3$ is H, $C_{1-5}$ alkyl or phenyl, or $NR^2R^3$ is a 5, 6 or 7-membered ring that may contain 1 to 3 double bonds and/or 1 or 2 further heteroatoms selected from N, O and S;

said enantiomer having the same absolute configuration at the asymmetric carbon atom (*) as that of the (−)-enantiomer of the compound of formula I wherein Q is H;

or a pharmaceutically-acceptable acid addition salt thereof.

2. A pure enantiomer or salt thereof according to claim 1, wherein Q is H, allyl, benzyl, $R^1CO$— or $R^2R^3N$—CO— wherein $R^1$ is $C_{1-5}$ alkyl, phenyl, 2,6-dimethylphenyl, 3- or 4-hydroxyphenyl or 3- or 4-alkanoyloxyphenyl, $R^2$ is $C_{1-5}$ alkyl, phenethyl, benzyl or phenyl, and $R^3$ is H or $C_{1-5}$ alkyl.

3. A pure enantiomer or salt thereof according to claim 2, wherein Q is H, $R^1CO$— or $R^2R^3N$—CO—.

4. A pure enantiomer according to claim 1, which is (−)-N-n-propyl-3-(3-hydroxyphenyl)piperidine or a pharmaceutically-acceptable acid addition salt thereof.

5. Use of a pure enantiomer or salt thereof according to any preceding claim, for the manufacture of a medicament having post-synaptic dopamine antagonist activity.

**Claims for the Contracting State: AT**

1. Use of a compound for the manufacture of a medicament having pre-synaptic dopamine agonist activity and post-synaptic dopamine antagonist activity, in which the compound is a pure enantiomer of a compound of formula I

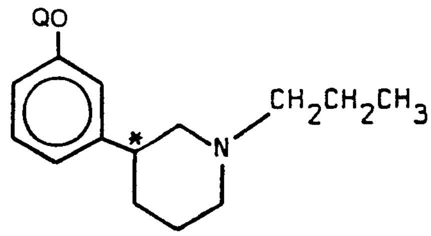

I

wherein Q is H, $R^1CO$—, $R^2R^3N$—CO—, allyl or benzyl;

wherein $R^1$ is $C_{1-17}$ aliphatic hydrocarbyl, phenyl, 2,6-dimethylphenyl, 3- or 4-hydroxyphenyl, 3- or 4-($C_{2-7}$ alkanoyloxy)phenyl, or —$CHR^6$—$NR^7R^8$ wherein $R^6$ is H, $C_{1-5}$ alkyl or phenyl, $R^7$ is H, $C_{1-5}$ alkyl, formyl, acetyl, benzoyl, methoxycarbonyl, phenoxycarbonyl or benzyloxycarbonyl, and $R^8$ is H or $C_{1-5}$ alkyl; and

either $R^2$ is H, $C_{1-5}$ alkyl, or a phenethyl, benzyl or phenyl group which may be mono- or di-ring-substituted by methyl, methoxy, hydroxy, nitro, cyano or a halogen atom, and $R^3$ is H, $C_{1-5}$ alkyl or phenyl, or $NR^2R^3$ is a 5, 6 or 7-membered ring that may contain 1 to 3 double bonds and/or 1 or 2 further heteroatoms selected from N, O and S;

said enantiomer having the same absolute configuration at the asymmetric carbon atom (*) as that of the (−)-enantiomer of the compound of formula I wherein Q is H;

or a pharmaceutically-acceptable acid addition salt thereof.

2. Use according to claim 1, wherein Q is H, allyl, benzyl, $R^1CO$— or $R^2R^3N$—CO— wherein $R^1$ is $C_{1-5}$ alkyl, phenyl, 2,6-dimethylphenyl, 3- or 4-hydroxyphenyl or 3- or 4-alkanoyloxyphenyl, $R^2$ is $C_{1-5}$ alkyl, phenethyl, benzyl or phenyl, and $R^3$ is H or $C_{1-5}$ alkyl.

3. Use according to claim 2, wherein Q is H, $R^1CO$— or $R^2R^3N$—CO—.

4. Use according to claim 1, in which the pure enantiomer is (−)-N-n-propyl-3-(3-hydroxyphenyl)piperidine or a pharmaceutically-acceptable acid addition salt thereof.

5. Use of a pure enantiomer or salt thereof according to any preceding claim, in which the activity is post-synaptic dopamine antagonist activity.

**Patentansprüche für die Vertragsstaaten: BE CH DE FR GB IT LU NL SE**

1. Ein reines Enantiomer einer Verbindung der Formel (I)

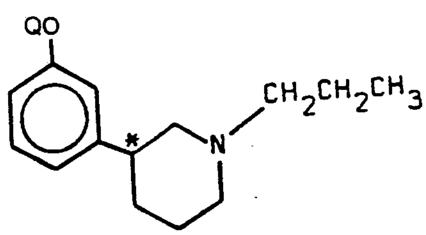

I

worin Q H, $R^1$—CO—, $R^2R^3N$—CO—, Allyl oder Benzyl bedeutet, worin $R^1$ $C_{1-17}$ aliphatisches Hydrocarbyl, Phenyl, 2,6-Dimethylphenyl, 3- oder 4-Hydroxyphenyl, 3- oder 4-($C_{2-7}$ Alkanoyloxy)phenyl oder —$CHR^6$—$NR^7R^8$, worin $R^6$ H, $C_{1-5}$ Alkyl oder Phenyl ist, $R^7$ H, $C_{1-5}$ Alkyl, Formyl, Acetyl, Benzoyl, Methoxycarbonyl, Phenoxycarbonyl oder Benzyloxycarbonyl ist und $R^8$ H oder $C_{1-5}$ Alkyl ist, bedeutet und entweder $R^2$ H, $C_{1-5}$ Alkyl oder eine Phenethyl-, Benzyl- oder Phenylgruppe, welche mit Methyl, Methoxy, Hydroxy, Nitro, Cyano oder einem Halogenatom mono- oder di-Ring substituiert sein kann und $R^3$ H, $C_{1-5}$ Alkyl oder Phenyl bedeutet oder $NR^2R^3$ einen 5-, 6- oder 7-gliedrigen Ring bedeutet, welcher 1 bis 3 Doppelbindungen und/oder 1 oder 2 weitere Heteroatome, gewählt aus N, O und S umfaßt, welches Enantiomer die gleiche absolute Konfiguration an dem asymmetrischen C-Atom (*) besitzt wie das (−)-Enantiomer der Verbindung der Formel I, in welcher Q H bedeutet; oder ein pharmazeutisch verträgliches Säureadditionssalz davon.

2. Ein reines Enantiomer oder ein Salz davon nach Anspruch 1, worin Q H, Allyl, Benzyl, $R^1CO$— oder $R^2R^3N$—CO—, worin $R^1$ $C_{1-5}$ Alkyl, Phenyl, 2,6-Dimethylphenyl, 3- oder 4-Hydroxyphenyl oder 3- oder 4-Alkanoyloxyphenyl ist, $R^2$ $C_{1-5}$ Alkyl, Phenethyl, Benzyl oder Phenyl ist, und $R^3$ H oder $C_{1-5}$ Alkyl ist, bedeutet.

3. Ein reines Enantiomer oder Salz davon nach Anspruch 2, worin Q H, $R^1$—CO— oder $R^2R^3$N—CO— bedeutet.

4. Ein reines Enantiomer nach Anspruch 1, welches (−)-N-n-Propyl-3-(3-hydroxyphenyl)piperidin oder ein pharmazeutisch verträgliches Säureadditionssalz davon ist.

5. Verwendung eines reinen Enantiomers oder eines Salzes davon, nach einem der vorhergehenden Ansprüche zur Herstellung eines Medikamentes, welches eine post-synaptische Dopaminantagonisten-Wirksamkeit besitzt.

**Patentansprüche für den Vertragsstaat: AT**

1. Verwendung einer Verbindung zur Herstellung eines Medikamentes, welches eine pre-synaptische Dopaminagonisten-Wirksamkeit und eine post-synaptische Dopaminantagonisten-Wirksamkeit besitzt, worin die Verbindung ein reines Enantiomer einer Verbindung der Formel (I)

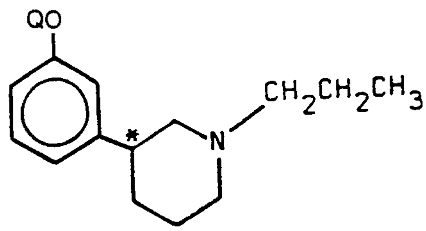

I

worin Q H, $R^1$—CO—, $R^2R^3$N—CO—, Allyl oder Benzyl bedeutet, worin $R^1$ $C_{1-17}$ aliphatisches Hydrocarbyl, Phenyl, 2,6-Dimethylphenyl, 3- oder 4-Hydroxyphenyl, 3- oder 4-($C_{2-7}$ Alkanoyloxy)phenyl oder —$CHR^6$—$NR^7R^8$, worin $R^6$ H, $C_{1-5}$ Alkyl oder Phenyl ist, $R^7$ H, $C_{1-5}$ Alkyl, Formyl, Acetyl, Benzoyl, Methoxycarbonyl, Phenoxycarbonyl oder Benzyloxycarbonyl ist und $R^8$ H oder $C_{1-5}$ Alkyl ist, bedeutet und entweder $R^2$ H, $C_{1-5}$ Alkyl oder eine Phenethyl-, Benzyl- oder Phenylgruppe, welche mit Methyl, Methoxy, Hydroxy, Nitro, Cyano oder einem Halogenatom mono- oder di-Ring substituiert sein kann und $R^3$ H, $C_{1-5}$ Alkyl oder Phenyl bedeutet oder $NR^2R^3$ einen 5-, 6- oder 7-gliedrigen Ring bedeutet, welcher 1 bis 3 Doppelbindungen und/oder 1 oder 2 weitere Heteroatome, gewählt aus N, O und S umfaßt, welches Enantiomer die gleiche absolute Konfiguration an dem asymmetrischen C-Atom (*) besitzt wie das (−)-Enantiomer der Verbindung der Formel I, in welcher Q H bedeutet; darstellt, oder ein pharmazeutisch verträgliches Säureadditionssalz davon.

2. Verwendung nach Anspruch 1, worin Q H, Allyl, Benzyl, $R^1$CO— oder $R^2R^3$N—CO—, worin $R^1$ $C_{1-5}$ Alkyl, Phenyl, 2,6-Dimethylphenyl, 3- oder 4-Hydroxyphenyl oder 3- oder 4-Alkanoyloxyphenyl ist, $R^2$ $C_{1-5}$ Alkyl, Phenethyl, Benzyl oder Phenyl ist, und $R^3$ H oder $C_{1-5}$ Alkyl ist, bedeutet.

3. Verwendung nach Anspruch 2, worin Q H, $R^1$—CO— oder $R^2R^3$N—CO— bedeutet.

4. Verwendung nach Anspruch 1, worin das reine Enantiomer (−)-N-n-Propyl-3-(3-hydroxyphenyl)piperidin oder ein pharmazeutisch verträgliches Säureadditionssalz davon ist.

5. Verwendung eines reinen Enantiomers oder eines Salzes davon, nach einem der vorhergehenden Ansprüche, worin die Wirksamkeit die post-synaptische Dopaminantagonisten-Wirksamkeit ist.

**Revendications pour les Etats contractants: BE CH DE FR GB IT LU NL SE**

1. Enantiomère pur d'un composé de formule I

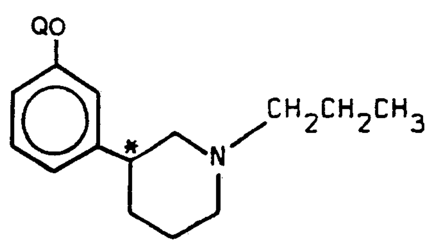

I

dans laquelle Q représente H, un groupe $R^1$—CO—, $R^2R^3$N—CO—, allyle ou benzyle;

$R^1$ est un radical hydrocarbyle aliphatique en $C_1$ à $C_{17}$, phényle, 2,6-diméthylphényle, 3- ou 4-hydroxyphényle, 3- ou 4-(alcanoyloxy en $C_2$ à $C_7$)phényle ou un radical —$CHR^6$—$NR^7R^8$ dans lequel $R^6$ représente H, un reste alkyle en $C_1$ à $C_5$ ou phényle, $R^7$ représente H, un reste alkyle en $C_1$ à $C_5$, formyle, acétyle, benzoyle, méthoxycarbonyle, phénoxycarbonyle ou benzyloxycarbonyle et $R^8$ représente H ou un reste alkyle en $C_1$ à $C_5$; et

$R^2$ représente H, un reste alkyle en $C_1$ à $C_5$ ou un groupe phénéthyle, benzyle ou phényle qui peut être monosubstitué ou disubstitué sur le noyau par un radical méthyle, méthoxy, hydroxy, nitro, cyano ou un atome d'halogène, et $R^3$ représente H, un reste alkyle en $C_1$ à $C_5$ ou phényle, ou bien $NR^2R^3$ est un noyau

pentagonal, hexagonal ou heptagonal qui peut contenir 1 à 3 doubles liaisons et/ou 1 ou 2 autres hétéroatomes choisis entre N, O et S;

ledit énantiomère ayant la même configuration absolue au niveau de l'atome asymétrique de carbone (*) que le (−)-énantiomère du composé de formule I dans laquelle Q représente H;

ou un sel d'addition d'acide pharmaceutiquement acceptable de ce composé.

2. Enantiomère pur ou sel de cet énantiomère suivant la revendication 1, dans lequel Q représente H, un groupe allyle, benzyle, $R^1CO—$ ou $R^2R^3N—CO—$, où $R^1$ est un radical alkyle en $C_1$ à $C_5$, phényle, 2,6-diméthylphényle, 3- ou 4-hydroxyphényle ou 3- ou 4-alcanoyloxyphényle, $R^2$ est un reste alkyle en $C_1$ à $C_5$, phénéthyle, benzyle ou phényle et $R^3$ représente H ou un reste alkyle en $C_1$ à $C_5$.

3. Enantiomère pur ou un sel de cet énantiomère suivant la revendication 2, dans lequel Q représente H, un groupe $R^1CO—$ ou $R^2R^3N—CO—$.

4. Enantiomère pur suivant la revendication 1, qui est la (−)-N-n-propyl-3-(3-hydroxyphényl)pipéridine ou un sel d'addition d'acide pharmaceutiquement acceptable de ce composé.

5. Utilisation d'un énantiomère pur ou d'un sel de cet énantiomère suivant l'une quelconque des revendications précédentes, pour la préparation d'un médicament doué d'activité d'antagonisation post-synaptique de la dopamine.

**Revendications pour l'Etat contractant: AT**

1. Utilisation d'un composé pour la préparation d'un médicament doué d'activité agoniste des récepteurs présynaptiques de dopamine et d'activité d'antagonisation des récepteurs post-synaptiques de dopamine, dans lequel le composé est un énantiomère pur d'un composé de formule I

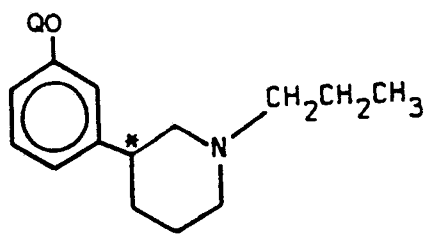

I

dans laquelle Q représente H, un groupe $R^1—CO—$, $R^2R^3N—CO—$, allyle ou benzyle;

$R^1$ est un radical hydrocarbyle aliphatique en $C_1$ à $C_{17}$, phényle, 2,6-diméthylphényle, 3- ou 4-hydroxyphényle, 3- ou 4-(alcanoyloxy en $C_2$ à $C_7$)phényle ou un radical $—CHR^6—NR^7R^8$ dans lequel $R^6$ représente H, un reste alkyle en $C_1$ à $C_5$ ou phényle, $R^7$ représente H, un reste alkyle en $C_1$ à $C_5$, formyle, acétyle, benzoyle, méthoxycarbonyle, phénoxycarbonyle ou benzyloxycarbonyle et $R^8$ représente H ou un reste alkyle en $C_1$ à $C_5$; et

$R^2$ représente H, un reste alkyle en $C_1$ à $C_5$ ou un groupe phénéthyle, benzyle ou phényle qui peut être monosubstitué ou disubstitué sur le noyau par un radical méthyle, méthoxy, hydroxy, nitro, cyano ou un atome d'halogène, et $R^3$ représente H, un reste alkyle en $C_1$ à $C_5$ ou phényle, ou bien $NR^2R^3$ est un noyau pentagonal, hexagonal ou heptagonal qui peut contenir 1 à 3 doubles liaisons et/ou 1 ou 2 autres hétéroatomes choisis entre N, O et S;

ledit énantiomère ayant la même configuration absolue au niveau de l'atome asymétrique de carbone (*) que le (−)-énantiomère du composé de formule I dans laquelle Q représente H;

ou un sel d'addition d'acide pharmaceutiquement acceptable de ce composé.

2. Utilisation suivant la revendication 1, dans lequel Q représente H, un groupe allyle, benzyle, $R^1CO—$ ou $R^2R^3N—CO—$, où $R^1$ est un reste alkyle en $C_1$ à $C_5$, phényle, 2,6-diméthylphényle, 3- ou 4-hydroxyphényle ou 3- ou 4-alcanoyloxyphényle, $R^2$ est un reste alkyle en $C_1$ à $C_5$, phénéthyle, benzyle ou phényle, et $R^3$ représente H ou un radical alkyle en $C_1$ à $C_5$.

3. Utilisation suivant la revendication 2, dans laquelle Q représente H, un groupe $R^1CO—$ ou $R^2R^3N—CO—$.

4. Utilisation suivant la revendication 1, dans laquelle l'énantiomère pur est la (−)-N-n-propyl-3-(3-hydroxyphényl)pipéridine ou un sel d'addition d'acide pharmaceutiquement acceptable de ce composé.

5. Utilisation d'un énantiomère pur ou d'un sel d'un énantiomère suivant l'une quelconque des revendications précédentes, dans laquelle l'activité est une activité d'antagonisation post-synaptique de dopamine.